# EUROPÄISCHE PATENTSCHRIFT

(11) **EP 4 097 212 B1**
(45) Veröffentlichungstag und Bekanntmachung des Hinweises auf die Patenterteilung: **08.04.2026**
(21) Anmeldenummer: 21704713.3
(22) Anmeldetag: 29.01.2021
(51) Int. Cl.: B01F 23/231, B01F 23/233, C12M 1/00, C12M 1/04

(54) **INTEGRALE BEGASUNGS- UND RÜHREINHEIT FÜR GAS-FLÜSSIG-REAKTOREN**
INTEGRAL GAS-INTRODUCTION AND STIRRING UNIT FOR GAS-LIQUID REACTORS
UNITÉ INTÉGRALE D'INTRODUCTION ET D'AGITATION DE GAZ POUR RÉACTEURS GAZ-LIQUIDE

(30) Priorität: 31.01.2020 DE 102020102420
(43) Veröffentlichungstag der Anmeldung: 07.12.2022
(73) Patentinhaber: Rheinisch-Westfälische Technische Hochschule (RWTH) Aachen, 52062 Aachen (DE)
(72) Erfinder: BONGARTZ, Patrick, 52074 Aachen (DE); MEYER, Moritz, 50259 Pulheim (DE); WESSLING, Matthias, 52074 Aachen (DE)
(74) Vertreter: Michalski Hüttermann & Partner Patentanwälte mbB
(86) Internationale Anmeldenummer: PCT/EP2021/052162
(87) Internationale Veröffentlichungsnummer: WO 2021/152128

(56) Entgegenhaltungen:
- EP-A1- 0 172 478
- DE-A1- 102005 053 333
- DE-A1- 102005 053 334
- DE-A1- 102006 008 687
- DE-B4- 102004 029 709
- DE-C1- 4 404 600

## Beschreibung

Die vorliegende Erfindung betrifft eine Begasungseinheit zum blasenfreien Eintrag eines Prozessgases in eine in einem Reaktor befindliche Flüssigkeit, wobei die Begasungseinheit mindestens umfasst:
- einen ersten und, von diesem beabstandet, einen zweiten Gasaufnahmeraum zur Aufnahme eines Prozessgases, wobei beide Gasaufnahmeräume über mindestens zwei flächig ausgebildete, gasführende Diffusionsmembranen aus voneinander beabstandeten und zumindest partiell gegeneinander fixierten Hohlfasern miteinander verbunden sind;
- eine Aufnahme für eine Gaszufuhr an mindestens einem der Gasaufnahmeräume;
- eine Aufnahme für eine Welle an mindestens einem der Gasaufnahmeräume;
wobei die Begasungseinheit zur Begasung der Flüssigkeit im Reaktor über die Gaszufuhraufnahme mit Prozessgas versorgbar, über die Aufnahme für die Welle in eine Rotationsbewegung versetzbar und über die Rotationsbewegung der Begasungseinheit in der Flüssigkeit eine Konvektionsströmung innerhalb des Reaktors ausbildbar ist. Des Weiteren betrifft die vorliegende Erfindung ein Verfahren zur Begasung einer Prozessflüssigkeit, einen Gas-Flüssig-Reaktor aufweisend eine erfindungsgemäße Begasungseinheit sowie die Verwendung einer erfindungsgemäßen Begasungseinheit zur Versorgung biologischer Kulturen mit Prozessgasen.

Die zuverlässige Herstellung wichtiger Grundstoffe über nachhaltigere Produktionsweisen steht heutzutage immer stärker im Fokus des öffentlichen Interesses. Dieser Ansatz betrifft dabei nicht nur die Herstellungsweise also solche, sondern erstreckt sich ebenfalls auf die Eigenschaften der verwendeten Stoffe nach Ablauf der geplanten Anwendungsdauer. Dies wird insbesondere daraus ersichtlich, dass für eine Vielzahl der in den vergangenen Jahrzehnten aus Erdöl synthetisierten Chemikalienklassen, nun verstärkt alternative und biologische Herstellungsvarianten entwickelt werden, welche neben einem ressourcen- und energieschonenderen Herstellprozess auch verbesserte chemische und biologische Eigenschaften, wie beispielsweise eine schnellere Abbaubarkeit, der hergestellten Substanzen liefern sollen.

Dieser Ansatz wird gerade für Tensid-Biomoleküle verfolgt, da diese Substanzen einen nachhaltigen Einfluss auf die Umwelt ausüben können und die Wertschöpfung, beispielsweise in kosmetischen oder pharmazeutischen Produkten, eine Kompensation der derzeit noch höheren Produktionskosten erlaubt. Eine umweltfreundlichere Herstellalternative basiert auf Fermentationsverfahren biologischer Systeme mit nachwachsenden Rohstoffen als Nährmedien und unter Sauerstoffeintrag. Nachteilig ist jedoch, dass in der Biosynthese von Proteinen und Tensiden üblicherweise eine starke Schaumbildung in den Fermentern auftritt, welche der Leistungsfähigkeit und dem gesamten Prozessablauf abträglich ist. Als bisheriges Mittel der Wahl für die Begasung dieser Systeme hat sich eine Blasenbegasung und anschließende Zerschlagung der Bläschen mittels Rührer oder aber der Einsatz von Anti-Schaummitteln herausgestellt. Dadurch sollte eine möglichst blasenfreie Fermentation ohne Verlust an Biomasse gewährleistet werden. Die Blasenbegasung inklusive Zerschlagung ist jedoch nachteilig, da weitere Regelparameter die Steuerung des Prozesses verkomplizieren und die mechanische Schaumzerstörung energetisch den Prozess verteuert. Schaumverhütungsmittel stellen keine nachhaltige Alternative dar, da die zusätzliche Aufreinigung im nachgeschalteten Prozessablauf die Prozesskosten stark beeinflusst. Insofern sind die bisherigen Fermentationsmethoden und die für diese genutzten Ausrüstungen verbesserungswürdig, um einen einfachen und reproduzierbaren Fermentationsprozess ohne Schaumbildung zu gewährleisten.

Auch in der Patentliteratur finden sich einige Ansätze zur blasenfreien Begasung von Fermentationsflüssigkeiten.

So beschreibt beispielsweise die DE 10 2006 008 687 A1 ein Verfahren zur Begasung von Flüssigkeiten, insbesondere in der Biotechnologie und insbesondere von Zellkulturen, mit einem Gasaustausch über eine oder mehrere eingetauchte Membranflächen wie Schläuche, Zylinder oder Module dadurch gekennzeichnet, dass diese Membranfläche eine beliebige, rotatorisch oszillierende Bewegung in der Flüssigkeit ausführt.

Des Weiteren offenbart die DE 44 046 00 C1 ein Verfahren zur blasenfreien Begasung von Mikroorganismen, die in einem Reaktor auf einem Trägermaterial immobilisiert sind, wobei das Trägermaterial mit den Mikroorganismen von einer wässrigen Lösung angeströmt wird, die vor dem Trägermaterial über Membranen, welche auf einer Seite mit einem sauerstoffhaltigen Gas beaufschlagt werden, mit Sauerstoff angereichert wurde.

In einem weiteren Patentdokument, der DE 41 42 502 A1, wird ein Verfahren zum blasenfreien Eintrag von Wasserstoff in wässrige Flüssigkeiten offenbart, wobei man den Wasserstoff über eine Membran in die wässrige Flüssigkeit einträgt, Das Verfahren zeichnet sich dadurch aus, dass man eine Membran verwendet, umfassend a) eine aus porösem Polymer gebildete Trägerstruktur, und b) mindestens eine Schicht aus porenfreiem Polymer, wobei die wässrige Flüssigkeit auf der Seite der Schicht aus porenfreiem Polymer mit der Membran in Kontakt steht.

Des Weiteren offenbart die DE 10 2005 053 334 A1 eine Vorrichtung zur Begasung von flüssigen Medien mittels Schlauchbegasung, darin enthaltene spezielle Schlauchmodule sowie die Verwendung der Vorrichtung zur Begasung von flüssigen Medien. Weitere Möglichkeiten zur Begasung von Prozessflüssigkeiten werden zudem in der DE 10 2006 008 687 A1 und der DE 10 2005 053 333 A1 offenbart.

Derartige aus dem Stand der Technik bekannte Lösungen können noch weiteres Verbesserungspotential bieten, insbesondere hinsichtlich der Effizienz in der Versorgung von Prozessflüssigkeiten mit Prozessgasen und insbesondere hinsichtlich einer verlässlichen Vermeidung einer Schaumbildung, auch für Systeme mit schwierigen Produkteigenschaften.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung eine Begasungseinheit und einen Gas-Flüssig-Reaktor mit dieser bereitzustellen, welche sich durch eine besonders effiziente und gleichmäßige Gasversorgung von Prozessflüssigkeiten, unter deutlicher Verringerung der Schaumbildung während des Eintrags, auszeichnet.

Die Lösung der Aufgabe erfolgt durch die Merkmale der jeweiligen unabhängigen Ansprüche, gerichtet auf die erfindungsgemäße Begasungseinheit, den erfindungsgemäßen Gas-Flüssig-Reaktor, das erfindungsgemäße Verfahren sowie die erfindungsgemäße Verwendung. Bevorzugte Ausgestaltungen der Erfindung sind in den Unteransprüchen, in der Beschreibung oder den Figuren beschrieben, wobei weitere in den Unteransprüchen oder in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Die Lösung der Aufgabe erfolgt erfindungsgemäß durch eine Begasungseinheit zum blasenfreien Eintrag eines Prozessgases in eine in einem Reaktor befindliche Flüssigkeit, wobei die Begasungseinheit mindestens umfasst:
- einen ersten und, von diesem beabstandet, einen zweiten Gasaufnahmeraum zur Aufnahme eines Prozessgases, wobei beide Gasaufnahmeräume über mindestens zwei flächig ausgebildete, gasführende Diffusionsmembranen aus voneinander beabstandeten und zumindest partiell gegeneinander fixierten Hohlfasern miteinander verbunden sind;
- eine Aufnahme für eine Gaszufuhr an mindestens einem der Gasaufnahmeräume;
- eine Aufnahme für eine Welle an mindestens einem der Gasaufnahmeräume;
wobei die Begasungseinheit zur Begasung der Flüssigkeit im Reaktor über die Gaszufuhraufnahme mit Prozessgas versorgbar, über die Aufnahme für die Welle in eine Rotationsbewegung versetzbar und über die Rotationsbewegung der Begasungseinheit in der Flüssigkeit eine Konvektionsströmung innerhalb des Reaktors ausbildbar ist.

Überraschenderweise hat sich gezeigt, dass sich über oben angegebenen Aufbau eine äußerst effiziente und belastbare Begasungs-Rührerkombination ergibt, welche sich für ein breites Spektrum an Begasungsanwendungen in unterschiedlichsten (Bio)Reaktoren eignet. Der Eintrag von Prozessgasen in eine Prozessflüssigkeit erfolgt in einem hohen Maße homogen und schonend, wobei durch die Kombination aus gleichzeitiger Begasungs- und Rührfläche über die Austauschfläche besonders große Mengen an Prozessgas gleichmäßig in die Flüssigkeit eingebracht werden können. Dies ergibt sich insbesondere daraus, dass Membran-Austauschflächen aus Hohlfasern eingesetzt werden, welche durch die konstante Bewegung innerhalb der Flüssigkeit immer von ausdiffundierenden Prozessgas aktiv befreit werden. Somit sind durch den erfindungsgemäßen Aufbau durch die Hohlfasermembranen zum einen insgesamt größere als die bisher bekannten Austauschflächen realisierbar und zum anderen werden diese noch effizienter als in den Stand der Technik Lösungen genutzt, da durch das simultan bewegte Abscheren der Gase an der Oberfläche der Membranen eine Diffusionsblockierung durch anhaftende Gasblasen gänzlich vermieden wird. Die Konstruktion ist durch die zentrale Zuführung des Prozessgases und die gleichmäßige Aufteilung dessen über einen Gasaufnahmeraum zudem so robust, dass über die fest verankerten Hohlfasern hohe Geschwindigkeiten und damit starke Konvektionsströmungen erzeugbar sind. Durch die Kombination aus Begasung und Rührung ist zudem sichergestellt, dass die Hohlfasern nicht nur mittelbar an- sondern aktiv durch die Prozessflüssigkeit überströmt werden. Es entstehen im Modul selbst keine unbewegten Toträume, welches zur Effizienz des Eintrages und zur Gleichmäßigkeit der Flüssigbegasung beiträgt. Zudem wird durch das Abscheren die Blasengröße klein gehalten. Somit lassen sich auch schwierige Fermentationsaufgaben mit lebenden Kulturen meistern, beispielsweise in der Produktion schaumfördernder Substanzen, da durch die Gleichmäßigkeit des Eintrags, die schiere Menge an Gas und die Kontrolle der Blasengröße durch das simultane Abscheren, eine Blasen- und/oder Schaumbildung im flüssigen Medium im hohen Maße verhindert wird. Die Begasungseinheit kann zudem aus beliebig vielen, seriell geschalteten Begasungseinheiten aufgebaut sein, welches die Reinigung und Sterilisation einzelner Module und die Auslegung innerhalb eines Up-Scalings auf größere Reaktorvolumina erleichtert.

Die erfindungsgemäße Begasungseinheit eignet sich zum blasenfreien Eintrag eines Prozessgases in eine in einem Reaktor befindliche Flüssigkeit. Prozessflüssigkeiten in Reaktoren können durch die erfindungsgemäße Begasungseinheit gleichzeitig gerührt und mit einem Prozessgas beaufschlagt werden. Dies bedeutet, dass kontinuierlich oder diskontinuierlich in zeitlichen Abständen in die Prozessflüssigkeit ein Prozessgas über die Begasungseinheit eingeleitet wird. Durch die Einleitung des Prozessgases wird die Konzentration des Prozessgases in der Flüssigkeit im Reaktor zumindest temporär und am Einspeisungsort erhöht. Mögliche Prozessgase können beispielsweise Sauerstoff, Stickstoff, Kohlendioxid, Kohlenmonoxid, Wasserstoff oder ähnliche Gase oder Mischungen daraus sein. Üblicherweise bilden die Prozessgase Edukte zur Durchführung weiterer chemischer Reaktionen in der Prozessflüssigkeit. Der erfindungsgemäße Aufbau ermöglicht das blasenfreie Einspeisen von Prozessgasen in Prozessflüssigkeiten. Unter blasenfrei im Sinne der Erfindung wird insbesondere verstanden, dass die Blasengröße des Prozessgases in einem Bereich liegt, in welchen die Blasen an der Oberfläche der Membranen mit bloßem Auge nicht oder nur sehr schwer sichtbar sind. Die Blasengröße kann beispielsweise in einer Größenordnung von wenigen Mikrometern liegen. Durch den erfindungsgemäßen Aufbau wird insbesondere verhindert, dass im Laufe der Prozesszeit sich an der Oberfläche der Prozessflüssigkeit ein Schaum bildet oder ablagert. Die Flüssigkeiten im Reaktor können beispielsweise wässrige Lösungen, Dispersionen oder Emulsionen sein. Der Eintrag ist aber nicht auf wässrige Systeme beschränkt. Es können auch nicht-wässrige, flüssige Systeme blasenfrei begast werden.

Die Begasungseinheit umfasst mindestens einen ersten und, von diesem beabstandet, einen zweiten Gasaufnahmeraum zur Aufnahme eines Prozessgases. Über eine Gaszuleitung kann ein Prozessgas in einen der beiden Gasaufnahmeräume eingespeist werden und sich in diesem gleichmäßig verteilen. Der Gasaufnahmeraum bildet ein Reservoir für das Prozessgas und kann im Gegensatz zur direkten Einspeisung in die Membranen auch mögliche Druckschwankungen kompensieren. Aus diesem Gasaufnahmeraum wird dann das Prozessgas über die Hohlfasermembranen in den zweiten Gasaufnahmeraum geleitet, wobei der Abstand zwischen beiden Aufnahmeräumen als Funktion der Reaktordimensionen, der Länge und mechanischen Stabilität der Hohlfasern, dem gewünschten Gaseintrag und der gewünschten Strömungsmechanik gewählt werden kann. Die Gasaufnahmeräume als solche können beispielsweise aus Metall oder Kunststoff gefertigt sein und eine rotationssymmetrische Symmetrie aufweisen. Die beiden Gasaufnahmeräume weisen zudem Aufnahmen für die Hohlfasermembranen auf, wobei die Aufnahmen eine unabhängige Fixierung jeder einzelnen Hohlfasermembran erlaubt. Die Aufnahmen für die Hohlfasermembranen können beispielsweise aus Nuten in der Oberfläche des Gasaufnahmeraum bestehen, in denen die Hohlfasermembranen mechanisch geklemmt oder geklebt und so gasdicht mit dem Gasaufnahmeraum verbunden werden können.

Die beiden Gasaufnahmeräume sind über mindestens zwei flächig ausgebildete, gasführende Diffusionsmembranen aus voneinander beabstandeten und zumindest partiell gegeneinander fixierten Hohlfasern miteinander verbunden. Die Einleitung von Prozessgasen in die Flüssigkeit erfolgt also nicht über die Gasaufnahmeräume, sondern über Hohlfasermembranen, welche gasleitend mit den beiden Gasaufnahmeräumen verbunden sind. Die Hohlfasern werden dabei nicht isoliert, d.h. einzeln, eingesetzt. Es werden mehrere Hohlfasern neben- oder hintereinander angeordnet, sodass aus der Anordnung der Hohlfasern eine flächige Membran ausgebildet wird. Zur weiteren Stabilisierung der Membran, können die einzelnen Hohlfasern auch durch weitere mechanische Mittel gegeneinander befestigt sein. So können die einzelnen Hohlfasern in Form eines Gewebes untereinander mit senkrecht oder annähernd senkrecht zu den Hohlfasern verlaufenden, nicht gasführenden Fäden oder Fasern stabilisiert sein. Beispielsweise können pro cm Hohlfasermembran bevorzugt eine, des Weiteren bevorzugt zwei, des Weiteren bevorzugt 3 Fixierungen in Form eines inerten Polymerfadens eingefügt werden, wobei der Faden alternierend über und unter den Hohlfasern durchgeführt wird und die Hohlfasern gegeneinander fixiert. Als Diffusionsmembranen oder Mikrofiltrationsmembranen eignen sich Membranen bei denen das Gas zuerst in die Membran hinein und nach Durchqueren der Hohlfaserhülle in die Prozessflüssigkeit diffundiert. Die Membranen können dicht oder porös sein, wobei die Porosität der Membran in einem Bereich liegt, dass bei einer ausreichenden Strömung eine Konzentrationspolarisation auf der Außenseite der Membran - und ein damit verbundenes Ausgasen des einzutragenden Gases in Form von Bläschen - verhindert wird. Mögliche Porengrößen nicht "dichter" Membranen können in einem Bereich von 20 nm bis zu 20 µm liegen. "Dichte" Diffusionsmembrane können einen mehrschichtigen Aufbau aufweisen. So können weitere Schichten das Eindringen der Prozessflüssigkeit oder gegebenenfalls die Rückdiffusion unerwünschter Gase in die Membran hinein verhindern. Des Weiteren sorgt der schichtweise Kompositaufbau dafür, dass auf einer mechanisch festen Schicht, wie beispielsweise poröses PMP, ein extrem dünner Film dichten Materials getragen werden kann, der die aktive Schicht darstellt, beispielsweise aus PMP, TMCTS oder PDMS/Silikon. Diese dünne, dichte Schicht ist für die Durchlässigkeit der verwendeten Gase maßgeblich. Eine möglichst dünne Schicht sorgt dabei für einen hohen Stoffeintrag. Das Verhältnis von Stoffdurchgang durch die Membran und deren Dicke verhält sich bei dichten Membranen reziprok. Eine dünne aktive Schicht ist jedoch meist mechanisch instabil und benötigt einen Träger, einen sogenannten Support. Ein möglichst poröser Support stellt dabei einen vernachlässigbaren Widerstand für den Gaseintrag in die Prozessflüssigkeit dar. Verwendbare Hohlfasern können beispielsweise aus PMP (Polymethylpenten) bestehen und einen Innendurchmesser von 0,2 mm und einen Außendurchmesser von 0,38 mm aufweisen. Möglich ist auch eine Verwendung mit PDMS (Polydimethylsiloxan)/Silikon Membranen mit einem Innendurchmesser von ca. 0,3 mm und einem Außendurchmesser von ca. 0,5 mm. Bevorzugt werden zur Ausbildung einer flächigen Membrananordnung aus den einzelnen Hohlfasern mehr als 50, des Weiteren bevorzugt mehr als 100, des Weiteren bevorzugt mehr als 500 Hohlfasern neben oder hintereinander angeordnet. Eine flächige Anordnung ergibt sich in den Fällen, in denen bevorzugt mehr als 40%, des Weiteren bevorzugt mehr als 50%, des Weiteren bevorzugt mehr als 60% der Fläche zwischen zwei voneinander beabstandeten, nicht direkt benachbarten Hohlfasern mit weiteren Hohlfasern belegt sind. Eine solche flächige Anordnung kann beispielsweise dadurch erreicht werden, indem Hohlfasern mit oben genannten Dimensionen in einem Abstand von größer oder gleich 0,05 mm und kleiner oder gleich 2,5 mm, des Weiteren bevorzugt von größer oder gleich 0,1 mm und kleiner oder gleich 1 mm voneinander beabstandet in den Gasaufnahmeraum fixiert werden. Das Gas strömt durch den Gasaufnahmeraum in die zu Membranen zusammengeschlossenen Hohlfasern und tritt je nach Triebkraft in die flüssige Phase auf der Außenseite der Faser aus.

Die Begasungseinheit weist mindestens zwei Diffusionsmembranen auf. Dies bedeutet, dass ausgehend vom Gasaufnahmeraum sich nicht nur eine Membran aus mehreren Hohlfasern mit einem durchgängigen Gasweg in die Prozessflüssigkeit hinein zum zweiten Gasaufnahmeraum erstreckt, sondern dass am ersten Gasaufnahmeraum mindestens zwei, voneinander beabstandete Membranen aus mehreren Hohlfasern angeordnet sind, welche über einen separaten Gasweg die Prozessflüssigkeit mit Gas versorgen können. Bevorzugt können mehr als 10, des Weiteren bevorzugt mehr als 50 und des Weiteren bevorzugt mehr als 100 einzelne Membranflächen aus angeordneten Hohlfasern vorliegen, die sich vom ersten Gasaufnahmeraum her in die Prozessflüssigkeit erstrecken.

Die Verbindung der einzelnen Hohlfasern mit dem Gasaufnahmeraum kann durch ein mechanisches Verklemmen der Hohlfasern in speziell dafür ausgestaltete Vorrichtungen am Gasaufnahmeraum oder aber durch ein Verkleben der Membranen mit dem Gas-Aufnahmeraum als solchen erfolgen. Vorzugsweise können die einzelnen Hohlfasermembranen mit dem Gasaufnahmeraum verklebt werden.

An einem der Gasaufnahmeräume befindet sich eine Aufnahme für eine Gaszufuhr. Der Gasaufnahmeraum kann über eine Zuleitung durch den Reaktor hindurch von außen mit dem Prozessgas versorgt werden. Dazu kann eine externe Gasquelle über ein Schlauch- oder Kapillarsystem in das Reaktorinnere zum Gasaufnahmeraum geführt werden. Dort kann der Schlauch oder die Kapillare an eine dafür ausgelegt Aufnahme des Gasaufnahmeraumes angeschlossen sein. Dies kann beispielsweise mittels eines Fittings gasdicht erfolgen. Es können als Fitting bevorzugt metrische flangelose flat bottom Verbindungselemente mit flangeloser Ferrule verwendet werden. Die Fittinge können beispielsweise aus Metall oder aus Kunststoff, beispielsweise PEEK bestehen. Der Anschluss kann zentrisch oder azentrisch am Gasaufnahmeraum angeordnet sein, wobei vorteilhafterweise der Anschluss auf einer Seite angeordnet ist, welche den Hohlfasermembranen auf dem Gasaufnahmeraum gegenüberliegt. Je nach Betriebsweise der Begasungseinheit können entweder nur ein oder aber beide Gasaufnahmeräume mit Aufnahmen für eine Gaszufuhr ausgestattet sein. Insbesondere ist es möglich, dass die Begasungseinheit in zwei Modi betrieben werden kann. Zum einen kann die Begasung im "cross-flow" oder alternativ in einem "dead-end" Modus erfolgen. Beim "dead-end" Modus wird Gas lediglich in einen Gasaufnahmeraum der Begasungseinheit gefördert. In dieser Fahrweise wird keine Aufnahme für eine Ableitung des nicht eingeleiteten Prozessgases, beispielsweise am anderen Gasaufnahmeraum, benötigt.

An einem der Gasaufnahmeräume befindet sich eine Aufnahme für eine Welle. Zum Eintrag der nötigen mechanischen Energie zur Bewegung der Begasungseinheit weist einer der Gasaufnahmeräume eine Vorrichtung zur Aufnahme einer Welle auf. Die Welle kann durch den Reaktor geführt und mit einem Antrieb oder einem Getriebe verbunden sein, welcher die Welle in eine Rotationsbewegung versetzen kann. Durch die rotierende Welle wird auch die Begasungseinheit in Rotation versetzt und kann über die flächigen Hohlfasermembranen die Prozessflüssigkeit bewegen. Die Bewegung der Welle und damit der Begasungseinheit kann in nur eine Richtung oder aber bevorzug in zwei Richtungen erfolgen. So kann eine konstante oder aber alternierende Drehrichtungen der Begasungseinheit mit unterschiedlichen Geschwindigkeiten realisiert werden.

Die Begasungseinheit ist zur Begasung der Flüssigkeit im Reaktor über die Gaszufuhraufnahme mit Prozessgas versorgbar, wobei über die Aufnahme für die Welle die Begasungseinheit in eine Rotationsbewegung versetzbar und über die Rotationsbewegung der Begasungseinheit in der Flüssigkeit eine Konvektionsströmung innerhalb des Reaktors ausbildbar ist. Durch die Gas- und die Wellenaufnahme können sowohl mechanische Energie wie auch Prozessgas auf die Begasungseinheit übertragen werden. Durch die Hohlfasermembranen wird Prozessgas in die Prozessflüssigkeit abgegeben und durch die Drehbewegung der Begasungseinheit, und hier eigentlich der flächigen Hohlfasermembranen, wird innerhalb der Flüssigkeit im Reaktor eine gerichtete Strömung erzeugt.

In einer bevorzugten Ausführungsform der Begasungseinheit können die Projektionen der Diffusionsmembranen auf die Aufnahmeräume eine Kreisbogengeometrie aufweisen. Zur Ausbildung eines gleichmäßigen Strömungsprofils innerhalb der Reaktorflüssigkeit und zur gleichmäßigen Überströmung der einzelnen Hohlfasern innerhalb der Begasungseinheit, hat es sich als besonders geeignet herausgestellt, dass die einzelnen Hohlfasern nicht in einer geraden Linie, sondern sowohl in längs- wie auch in Querrichtung voneinander entfernt auf dem Gasaufnahmeraum angeordnet werden. Es ergibt sich im Rahmen der flächigen Ausgestaltung der Membranen somit keine gerade, sondern eine gebogene Fläche aus angeordneten Hohlfasern. Neben der Verbesserung und Vergleichmäßigung des Strömungsprofils kann diese Ausgestaltung auch insbesondere dazu beitragen, dass der Gaseintrag in die Flüssigkeit durch ein gleichmäßiges Abscheren der Gasblasen von der Hohlfasermembranoberfläche verbessert wird. Insbesondere kann auch das Auftreten größerer Gasblasen auf der Oberfläche der Membranen verzögert oder ganz verhindert werden. Eine mögliche Ausgestaltung der Kreisbogengeometrie ist in den Figuren dargestellt. Bevorzugt kann der Kreisbogen eine Krümmung größer oder gleich 1 m⁻¹ und kleiner oder gleich 100 m⁻¹, des Weiteren bevorzugt von größer oder gleich 5 m⁻¹ und kleiner oder gleich 70 m⁻¹ aufweisen.

Innerhalb einer weiter bevorzugten Ausgestaltung der Begasungseinheit kann die Aufnahme für die Gaszufuhr und die Aufnahme für die Welle an nur einem Gasaufnahmeraum angeordnet sein sind. Zur Vergleichmäßigung des Strömungsprofils im Reaktor hat es sich als besonders vorteilhaft herausgestellt, dass die Gasaufnahme und der Anschluss für die Antriebswelle mittig an nur einem Gasaufnahmeraum angeordnet sind. Weiterhin bevorzugt können dabei sowohl die Gas- wie auch die Wellenaufnahme zusammen ausgeführt sein, beispielsweise in Form einer Hohlwelle, sodass beide Aufnahmen innerhalb eines Anschlusses an der Begasungseinheit liegen. Dies kann zur Verringerung der Anzahl mechanischer Komponenten an der Begasungseinheit beitragen. Zudem kann dieser Kombianschluss bevorzugt mittig an der Begasungseinheit ausgeführt sein. Bevorzugt kann die Begasungseinheit über nur eine kombinierte Aufnahme an einem der Gasaufnahmeräume gleichzeitig mit Prozessgas und der nötigen kinetischen Energieversorgt werden. Dies kann das Strömungsprofil der Begasungseinheit besonders homogen halten und den apparativen Anschlussaufwand verringern. Letzteres kann auch zu einer besseren Reinig- und Sterilisierbarkeit der Begasungseinheit beitragen.

Innerhalb eines weiter bevorzugten Aspektes der Begasungseinheit können die beiden Gasaufnahmeräume jeweils zylindrisch ausgebildet und über eine oder mehrere mechanische Stützen miteinander verbunden sein. Eine rotationssymmetrische, zylindrische Geometrie hat sich zur Ausbildung eines möglichst effizienten Konvektionsstromes innerhalb der meisten Reaktorgeometrien als besonders vorteilhaft herausgestellt. Über diese Ausgestaltung der Gasaufnahmeräume können sowohl innerhalb der Begasungseinheit, wie auch im Reaktor selbst, reproduzierbar sehr gleichmäßige und auch starke Strömungen induziert werden, welches zu einer besonders guten Versorgung der Prozessflüssigkeit mit Prozessgasen beiträgt. Neben der Fixierung der relativen Position der beiden Gasaufnahmeräume über die Hohlfasermembranen gegeneinander, hat es sich zudem als günstig herausgestellt, dass die beiden Gasaufnahmeräume über eine oder mehrere mechanische Stützen in ihrer relativen Lage gegeneinander fixiert werden. Dies Maßnahme kann den Gleichlauf und ungewollte Schwingungen der Begasungseinheit unter hohen Drehzahlen reduzieren. Bevorzugt kann die Stütze durch den Mittelpunkt der beiden zylindrisch ausgestalteten Gasaufnahmeräume geführt sein. Eine solche Ausführungsform kann das Strömungsprofil innerhalb der Begasungseinheit, und hier insbesondere zwischen den einzelnen Hohlfasermembranen, verbessern.

Nach einer bevorzugten Charakteristik der Begasungseinheit kann zwischen den beiden Gasaufnahmeräumen an der mechanischen Stütze mindestens eine Haltescheibe angeordnet sei, welche dazu eingerichtet ist die Diffusionsmembranen mechanisch zu halten. Zur Ausbildung eines spiegelsymmetrischen Konvektionsstromes innerhalb einer großen Zahl unterschiedlicher Reaktorgeometrien, hat es sich als besonders günstig herausgestellt, dass zwischen den beiden Gasaufnahmeräumen an der Stütze eine Haltescheibe angeordnet ist, welche die Hohlfasermembranen mechanisch kontaktiert. Die Hohlfasermembranen können durch das mechanisch Halten durch die Haltescheibe entweder nur "lose" fixiert oder aber auch durch die Haltescheibe aus der Falllinie gestreckt oder verdreht werden. Im ersteren Fall können über das Halten der Hohlfasermembranen auch größere mechanische Kräfte, beispielsweise durch höhere Umlaufgeschwindigkeiten der Begasungseinheit, realisiert werden, ohne dass die Gefahr besteht, dass die Membranen beschädigt werden. Es können auch insgesamt fragilere Hohlfasern eingesetzt werden. Neben der mechanischen Aufgabe des Haltens der Membranen, kann aber auch die erzielbare Strömungsgeometrie mittels der Haltescheibe beeinflusst werden. Die einzelnen Hohlfasermembranen können gezielt aus der durch die Anbindung an die Gasaufnahmeräume vorgegebenen Geometrie ausgelenkt oder verdreht werden. Diese Auslenkung ändert die flächige Geometrie der Membranen und kann spezielle Konvektionsmuster in der Flüssigkeit hervorrufen. Auf diese Art und Weise können die Membranen auf spezielle Begasungsaufgaben und Reaktorgeometrien eingestellt werden.

In einer bevorzugten Ausgestaltung der Begasungseinheit kann die mechanische Stütze dazu eingerichtet sein, Prozessgas aus den Gasaufnahmeräumen zu transportieren. Zum Erhalt einer möglichst kompakten Bauform der Begasungseinheit mit einer verbesserten Versorgung der Einheit im Cross-Flow-Betrieb hat es sich als besonders geeignet herausgestellt, dass die mechanischen Stützen in Form von Hohlwellen ausgestaltet sind, welche ebenfalls Prozessgas zu- und von den Gasaufnahmeräumen wegleiten können.

In einer weiter bevorzugten Ausführungsform der Begasungseinheit kann das Flächenverhältnis aus gesamter Hohlfaser-Querschnittsfläche zur Querschnittsfläche des Gasaufnahmeraumes größer oder gleich 5 % und kleiner oder gleich 45% betragen. Mittels des vorgeschlagenen Aufbaus können kompakte Begasungseinheiten bereitgestellt werden, welche im Vergleich zu dem im Stand der Technik aufgeführten Lösungen eine deutlich größere Prozessgasaustauschfläche aufweisen. Diese großen Austauschflächen zeigen eine nur geringe Neigung zur Blasenbildung und bilden zudem noch ein günstigeres Strömungsverhalten der Begasungseinheit aus. Die gesamte Hohlfaser-Querschnittsfläche ergibt sich dabei aus dem Querschnitt einer einzelnen Hohlfaser multipliziert mit der Menge der am Gasaufnahmeraum angeordneten Fasern. Die Querschnittsfläche des Gasaufnahmeraumes ergibt sich aus der Fläche der mit Prozessgas versorgten Fläche des Gasaufnahmeraumes. In dem Fall, dass der Gasaufnahmeraum eine mittlere oder äußere Fläche aufweist, an welcher mangels Prozessgasversorgung keine Hohlfasermembranen angebracht werden können, trägt diese Fläche nicht mit zum oben genannten Verhältnis bei, obwohl der Gasaufnahmeraum diese Fläche umfasst. Innerhalb einer bevorzugten Ausführungsform kann das Flächenverhältnis größer oder gleich 7,5% und kleiner oder gleich 20 %, des Weiteren bevorzugt größer oder gleich 10% und kleiner oder gleich 15 % betragen. Innerhalb dieser Verhältnisse aus Begasungs-Austauschfläche und Gasaufnahmeraum können große Prozessgasmengen unter homogenen Strömungsbedingungen eingebracht werden.

In einer weiter bevorzugten Ausführungsform der Begasungseinheit kann die Packungsdichte der Diffusionsmembranen bezogen auf das Volumen der Begasungseinheit, ausgedrückt als Oberfläche der Hohlfasern dividiert durch das Volumen der Begasungseinheit, größer oder gleich 0,1 cm⁻¹ und kleiner oder gleich 7,5 cm⁻¹ betragen. Die Gesamtoberfläche der Hohlfasern kann dabei über die Anzahl und die frei für Prozessflüssigkeiten zugängliche Oberfläche der Hohlfasern berechnet werden. Das Gesamtvolumen der Begasungseinheit ergibt sich im Falle zylindrischer oder nicht-zylindrischer Geometrien über das zwischen den Gasaufnahmeräumen für Prozessflüssigkeiten zugängliche Volumen der Begasungseinheit. Über die erfindungsgemäße Ausgestaltung lassen sich sehr hohe aktive Begasungsflächen auf kleinem Raum unterbringen, welches neben einem hohen Prozessgasstrom in Kombination mit dem simultanen Rühren, auch zur effizienten Versorgung sehr hoher Reaktorvolumina beitragen kann. Bevorzugt kann das Verhältnis auch größer oder gleich 0,25 cm⁻¹ und kleiner oder gleich 6 cm⁻¹, des Weiteren bevorzugt größer oder gleich 0,5 cm⁻¹ und kleiner oder gleich 3 cm⁻¹ betragen.

Des Weiteren erfindungsgemäß ist ein Verfahren zur Begasung einer Prozessflüssigkeit innerhalb eines Reaktors, wobei der Gaseintrag über eine erfindungsgemäße Begasungseinheit erfolgt. Der Verfahrensschritt der Begasung einer Prozessflüssigkeit über eine erfindungsgemäße Begasungseinheit kann mehrere Verfahrensvorteile aufweisen. Es können in diesem Verfahrensschritt große Mengen an Prozessgas homogen im Flüssigkeitsvolumen bereitgestellt werden, wobei sich durch die Kombination aus Rühren und Begasen ein Eintrag ohne oder mit nur sehr kleinen Blasengrößen realisieren lässt. Durch den direkten Kontakt mit der Fluidgrenze und durch das unmittelbare Abscheren der Blasen von den Membranflächen werden Diffusionshemmungen durch eine Konzentrationspolarisation an der Oberfläche der Hohlfasern vermieden und, im Gegensatz zu stationären Anordnungen, wird durch die gleichmäßige Bewegung sämtlicher Fasern ein größeres Flüssigkeitsvolumen im Reaktor versorgt. Insbesondere durch die gesteuerte Ausbildung einer Konvektion innerhalb dieses Verfahrensschrittes, können Totzonen im Reaktor und innerhalb der Begasungseinheit vermieden werden.

In einer bevorzugten Ausführungsform des Verfahrens kann die Umlaufgeschwindigkeit der Membranfläche am äußersten Rand der Begasungseinheit größer oder gleich 0,1 m/s und kleiner oder gleich 5 m/s betragen. Mittels des erfindungsgemäßen Aufbaus können auch an sich mechanisch instabile Diffusionsmembranen unter hohen Scherraten in der Flüssigkeit betrieben werden. Ohne durch die Theorie gebunden zu sein ergibt sich dies dadurch, dass die beabstandeten Hohlmembranflächen durch- oder überströmt werden und so nur einen Teil des Bewegungsimpulses der Flüssigkeit aufnehmen. Es ergibt sich vorteilhafterweise ein Abstreifen des ausdiffundierten Prozessgases und eine Verringerung der mechanischen Belastung. Die Geschwindigkeit der Begasungseinheit kann dabei über die Ausmaße der Fläche der Gasaufnahmeräume und über die Rotationsgeschwindigkeit der Begasungseinheit als solche eingestellt werden. Aus dem Bezug auf die Hohlfaser, welche am weitesten vom Mittelpunkt der Begasungseinheit entfernt liegt, ergibt sich eine maximale Umlaufgeschwindigkeit für die Hohlfasern. Weiter im Inneren der Begasungseinheit liegende Hohlfasern weisen demzufolge eine geringe Umlaufgeschwindigkeit auf. Weiter bevorzugt kann die Umlaufgeschwindigkeit der Membranfläche am äußersten Rand der Begasungseinheit größer oder gleich 0,25 m/s und kleiner oder gleich 4 m/s, des Weiteren bevorzugt größer oder gleich 0,5 m/s und kleiner oder gleich 3 m/s betragen.

Weiterhin erfindungsgemäß ist ein Gas-Flüssig-Reaktor, wobei der Gas-Flüssig-Reaktor mindestens eine äußere Reaktorhülle, eine Antriebseinheit, eine Gaszufuhr und eine erfindungsgemäße Begasungseinheit umfasst. Der erfindungsgemäße Reaktor ist ein Gas-Flüssig-Reaktor zur blasenfreien Begasung einer Prozessflüssigkeit mit einem Prozessgas. Ein Gas-Flüssig-Reaktor im Sinne der Erfindung wird durch eine äußere Hülle begrenzt, welche beispielsweise aus Stahl oder Glas bestehen kann, welche in ihren Inneren einen Raum ausbildet, welcher in unterschiedlichen Füllhöhen mit einer Prozessflüssigkeit gefüllt werden kann. Insbesondere ist auch der Einsatz in Einmal-Reaktoren aus Kunststoffen wie PP, PC, PET, LDPE, EVA, PVDC und Verbundsystemen aus diesen Kunststoffen vorteilhaft möglich. Die Prozessflüssigkeit kann neben der Flüssigkeit als solche auch weitere Bestandteile wie beispielsweise Edukte, suspendierte Zellen bzw. Organismen, Salze, pH-Regulatoren oder weitere Substanzen aufweisen. Die Prozessflüssigkeit kann beispielsweise in Form einer wässrigen Lösung, Dispersion oder Emulsionen ausgebildet sein. Die Begasungseinheit kann über die Gasaufnahme mit einer Zuleitung für das Prozessgas verbunden sein. Das Prozessgas kann dabei in einem weiteren Reservoir, wie beispielsweise einer Gasflasche vorliegen und über Regelventile gesteuert abgegeben werden. Durch eine Kontrolleinheit kann der Gasfluss und die Zusammensetzung des Gases zur Begasungseinheit eingestellt werden. Beispielsweise durch ein Gasventil kann ein Transmembrandruck zwischen Innenseite der Membranen der Begasungseinheit und der Prozessflüssigkeit, beispielsweise in Form einer Fermenterbrühe, auf der äußeren Seite der Membran eingestellt werden. Der Gaseintrag in die Brühe skaliert üblicherweise proportional mit dem Transmembrandruck. Wird die Membran kontinuierlich mit Gas auf der Innenseite über-/durchströmt, ist der Betriebsmodus "cross-flow" eingestellt. Eine nur zeitweilige Überströmung des Membranlumens im Sinne einer "Spülung" ist auch möglich, dafür wird bei einer "dead-end"-Betriebsweise zwischenzeitlich ein Gasventil geöffnet. Dies ist insbesondere dann sinnvoll, wenn Gase aus dem Prozess, bei klassischen aeroben Fermentationen zum Beispiel CO₂, entlang des Konzentrationsgradienten in die Membran gelangen und sich dort konzentrieren. Diese Aufkonzentration kann zu der Minderung der Gesamtleistung des Gaseintrags führen, da der Partialgasdruck des einzutragenden Gases verringert wird und die Triebkraft weniger ist. Zudem kann auch Kondensationswasser, dass durch die Poren in die Membran gelangt ist, so zügig/stoßweise ausgetragen werden. Ein besonders hervorzuhebender Vorteil der "dead-end" Begasung ist, das stöchiometrisch nur die Gasmoleküle "eingetragen" also verwendet werden, die auch im Prozess metabolisiert werden. Dies ist in Hinblick auf die Wirtschaftlichkeit eines (Bio-)Prozesses relevant. Neben diesen Mindestbestandteilen kann der Reaktor natürlich auch noch weitere Einbauten aufweisen. So ist es beispielsweise möglich, dass im Reaktorinneren noch weitere Bestandteile wie beispielsweise Sensoren, Zu- und Ableitungen, Heizungen und/oder Kühlvorrichtungen angeordnet sind. Die Heiz- oder Kühlvorrichtungen können auch außerhalb des Reaktors angeordnet sein.

In einer bevorzugten Ausführungsform des Gas-Flüssig-Reaktors kann der Reaktor neben der Begasungseinheit keine weitere Rühreinheit aufweisen. Zur Ausbildung eines besonders effizienten Strömungsprofils mit einer homogenen Durchmischung auch der im Inneren der Begasungseinheit befindlichen Prozessflüssigkeit, hat es sich als geeignet herausgestellt, dass der Reaktor keine weiteren aktiv beweglichen Vorrichtungen zur Erzeugung einer gerichteten Strömung in der Prozessflüssigkeit aufweist. In diesen Fällen können die Hohlfasern besonders gezielt von der Prozessflüssigkeit umströmt und ein Großteil der aus diesen ausdiffundieren Prozessgasen abgeschert werden. Weitere aktive Rühreinheiten könnten die Symmetrie der erreichbaren Konvektion stören und zu unterschiedlichen Gaseintragsmengen pro Volumeneinheit Prozessflüssigkeit führen.

Innerhalb einer weiter bevorzugten Ausgestaltung des Gas-Flüssig-Reaktors kann zwischen der Reaktorhülle und einem Gasaufnahmeraum mindestens ein Strömungsbrecher angeordnet sein. Neben einer sehr symmetrischen Ausgestaltung des Konvektionsstromes kann es sinnvoll sein, die über die erfindungsgemäße Begasungs- und Rühreinheit induzierte Konvektion durch Strömungsbrecher in bestimmten Bereichen des Reaktors umzuleiten. Dies kann zu einer besseren Adaptierbarkeit auf spezifische Reaktorgeometrien beitragen. Die Strömungsbrecher können dabei sowohl zwischen der Begasungseinheit und den Reaktorwänden, wie auch zwischen der Begasungseinheit und dem Reaktorboden sowie -Deckel angebracht sein. Besonders bevorzugt kann mindestens ein Strömungsbrecher zwischen Begasungseinheit und Reaktordeckel angebracht sein. Dieser Strömungsbrecher kann zudem scheibenförmig ausgestaltet sein. Dieser Strömungsbrecher kann insbesondere bei hohen Austauschflächen und hohen Umlaufgeschwindigkeiten der Begasungseinheit dazu beitragen, dass eine unkontrollierte Aufnahme von Gas aus dem Kopfgasraum des Reaktors unterbunden wird. Insbesondere wird eine Konusbildung der Prozessflüssigkeit in Richtung Begasungseinheit effektiv unterbunden. Bevorzugt kann ein scheibenförmiger Strömungsbrecher in einer Höhe eingebracht werden, welche größer oder gleich ¼ und kleiner oder gleich ¾ des Abstandes obere Kante der Begasungseinheit bis zum Flüssigkeitsspiegel beträgt.

Weiterhin erfindungsgemäß ist die Verwendung eines erfindungsgemäßen Gas-Flüssig-Reaktors zur Versorgung in einer Prozesslösung suspendierter oder an dem Reaktorinneren oder an der Begasungseinheit anhaftender biologischer Kulturen mit Prozessgasen. Biologische Kulturen, beispielsweise in Form von Bakterien oder Pilzen, können in Bioreaktoren prinzipiell über zwei unterschiedliche Arten vermehrt werden. Zum einen können die Organismen in Lösung, beispielsweise in Form einer Suspension, oder aber an Oberflächen anhaftend vorliegen. Die Kultivierung als Biofilm kann prinzipiell an den Wandungen des Reaktors oder an der erfindungsgemäßen Begasungseinheit erfolgen. Letzteres ist erfindungsgemäß bevorzugt, da unter den dynamischen Begasungs- und Rührbedingungen eine gleichmäßigere Versorgung mit Nährstoffen gewährleistet werden kann. Insofern können höhere Ausbeuten und schnellere Umsetzungen erzielt werden. Der erfindungsgemäße Reaktoraufbau hat sich insbesondere zur Vermehrung von Mikroorganismen in einem wässrigen Fermentationsmedium als geeignet herausgestellt. Durch das erfindungsgemäße Reaktordesign lassen sich gezielt und reproduzierbar adaptierbare Gasmengen in das Reaktionsmedium einbringen, wobei die Durchmischung des gesamten Reaktorvolumens durch die Begasungseinheit nur zu einem sehr geringen Teil behindert wird. Es ergeben sich keine Totzonen mit geringerer Durchmischung und der Prozessgasbedarf, beispielsweise Sauerstoff, kann gleichmäßig und schnell im gesamten Volumen der Prozessflüssigkeit verteilt werden. Mikroorganismen sind dabei lebende Organismen, welche innerhalb des Reaktors vermehrt werden können. Für die weiteren Vorteile des erfindungsgemäßen Verfahrens wird explizit auf die Vorteile der erfindungsgemäßen Begasungseinheit Bezug genommen.

Weiterhin erfindungsgemäß ist die Verwendung des Gas-Flüssig-Reaktors zur Versorgung von Bakterien in einem Nährmedium mit Sauerstoff. Der erfindungsgemäße Reaktor kann insbesondere dazu genutzt werden, Bakterien innerhalb eines Nährmediums mit Sauerstoff zu versorgen. Gerade Bakterien zeigen als Funktion ihrer Wachstumsphase in Nährmedien einen unterschiedlichen Prozessgasbedarf. Die homogene Dispergierung des eingetragenen Sauerstoffs und die gleichmäßige Verteilung ist in diesen Fällen besonders schwierig, da das Begasungssystem ausreichende Reserven aufweisen muss, um sowohl geringe wie auch hohe Sauerstoffmengen gezielt in das Nährmedium zu bringen. In diesen Fällen muss beispielsweise der Transmembrandruck hoch flexibel sein, sodass geringe Mengen reproduzierbar und hohe Mengen ohne Blasenbildung einbringbar sind. Ein Beispiel für ein einsetzbares Bakterium ist *Pseudomonas putida* (*P. putida*), ein gramnegatives Stäbchenbakterium, das sowohl im Wasser, Boden oder an Pflanzen vorkommt. In Deutschland ist der Laborstamm *P. putida* KT2440 als S1-Organismus eingestuft und besitzt den GRAS-Status. Für die industrielle Biotechnologie ist *P. putida* KT2440 ein äußerst interessanter Organismus, da er neben einem vielseitigen Stoffwechsel, auch eine ausgeprägte Toleranz gegenüber organischen Lösungsmitteln besitzt. Des Weiteren ist *P. putida* ein beliebter Organismus für die heterologe Expression von Genen und besitzt eine hohe Wachstumsrate auf Glukose.

Innerhalb eines weiter bevorzugten Aspektes der Verwendung können die biologischen Kulturen dazu eingerichtet sein schaumbildende Substanzen herzustellen. Insbesondere schaumbildende Substanzen und unter diesen insbesondere Biotenside lassen sich in herkömmlichen Reaktoren besonders schwer fermentieren, da diese Tenside naturgemäß zu einer besonders starken Schaumbildung beitragen. Als ein Beispiel für ein Biotensid können Rhamnolipide dienen, welche durch Biokatalysatoren hergestellte grenzflächenaktive Moleküle darstellen. Diese Biotenside haben den ökologischen Vorteil, dass sie im Gegensatz zu erdölbasierten Tensiden biologisch schnell verstoffwechselt werden können und somit umweltverträglicher sind. Diese lassen sich besonders vorteilhaft in dem erfindungsgemäßen Reaktor mit der erfindungsgemäßen Begasungseinheit produzieren. Es existieren noch weitere Biotenside, die von verschiedenen Mikroorganismen hergestellt werden. Dazu gehören unter anderem Sophorolipide, die von Hefen produziert werden. Sophorolipide gehören zu den Glykolipiden. Des Weiteren gibt es Biotenside die zur Klasse der Lipopeptide zählen, wie zum Beispiel Surfactin das von *Bacillus subtilis* produziert wird. Surfactin wird vor allem im medizinischen Bereich angewendet. Diese lassen sich sehr schaumarm oder schaumfrei mit der erfindungsgemäßen Begasungseinheit herstellen.

In einer bevorzugten Ausführungsform der Verwendung kann die Membranfläche der Diffusionsmembranen bezogen auf das Reaktorfüllvolumen, ausgedrückt als cm² Membranfläche dividiert durch cm³ Reaktorfüllvolumen, größer oder gleich 0,05 cm⁻¹ und kleiner oder gleich 1,0 cm⁻¹ betragen. Insbesondere zur Zellzüchtung in Bioreaktoren und zur Synthese von Biomolekülen hat sich oben angegebenes Verhältnis von Membranfläche zu Reaktorvolumen als besonders vorteilhaft herausgestellt. Mittels dieses Verhältnisses lassen sich die unterschiedlichen Wachstumsphasen mit deutlich unterschiedlichen Mengen an Biomasse ausreichend mit Prozessgas versorgen, wobei auch am Anfang der Zellvermehrung eine ausreichende Steuerbarkeit des Eintrags an Prozessgas gegeben ist. Bedarfsgerecht kann mittels dieser Ausgestaltung eine sehr steuerbare und in späteren Wachstumsphasen bei ausreichend kleinen Transmembrandrücken, eine angepasste Menge an Prozessgas eingespeist werden.

Weitere Vorteile und vorteilhafte Ausgestaltungen der erfindungsgemäßen Gegenstände werden durch die Figuren veranschaulicht und in den nachfolgenden Beispielen erläutert. Dabei ist zu beachten, dass die Figuren nur beschreibenden Charakter haben und nicht dazu gedacht sind, die Erfindung in einer Form einzuschränken.

Es zeigt die Figur eine
1 eine erfindungsgemäße Ausgestaltung eines Gasaufnahmeraums in der Aufsicht;
2 eine weitere erfindungsgemäße Ausgestaltung eines Gasaufnahmeraums in der Aufsicht;
3 eine erfindungsgemäße Ausgestaltung einer Begasungseinheit in der Seitenansicht;
4 eine erfindungsgemäße Ausgestaltung einer Begasungseinheit in der Frontansicht;
5 einen schematischen Schnitt durch eine erfindungsgemäße Begasungseinheit;
6 einen Schnitt durch eine erfindungsgemäße Begasungseinheit;
7 einen schematischen Schnitt durch einen erfindungsgemäßen Reaktor mit erfindungsgemäßer Begasungseinheit;
8 eine schematische Frontansicht eines erfindungsgemäßen Reaktors mit erfindungsgemäßer Begasungseinheit inklusive eines möglichen, konvektiven Strömungsprofils;
9 eine schematische Frontansicht einer erfindungsgemäßen Begasungseinheit aus zwei seriell geschalteten Begasungseinheiten;
10 eine schematische Seitenansicht einer erfindungsgemäßen Begasungseinheit aus zwei seriell geschalteten Begasungseinheiten;
11 eine schematische Frontansicht einer erfindungsgemäßen Begasungseinheit aus zwei seriell geschalteten Begasungseinheiten mit Medienversorgung;
12 eine schematische Frontansicht eines erfindungsgemäßen Reaktors mit zwei seriell geschalteten, erfindungsgemäßen Begasungseinheiten mit Medienversorgung;
13 eine schematische Frontansicht eines erfindungsgemäßen Reaktors mit zwei seriell geschalteten, erfindungsgemäßen Begasungseinheiten inklusive eines möglichen, konvektiven Strömungsprofils;
14 eine schematische Explosionsansicht einer erfindungsgemäßen Begasungseinheit.

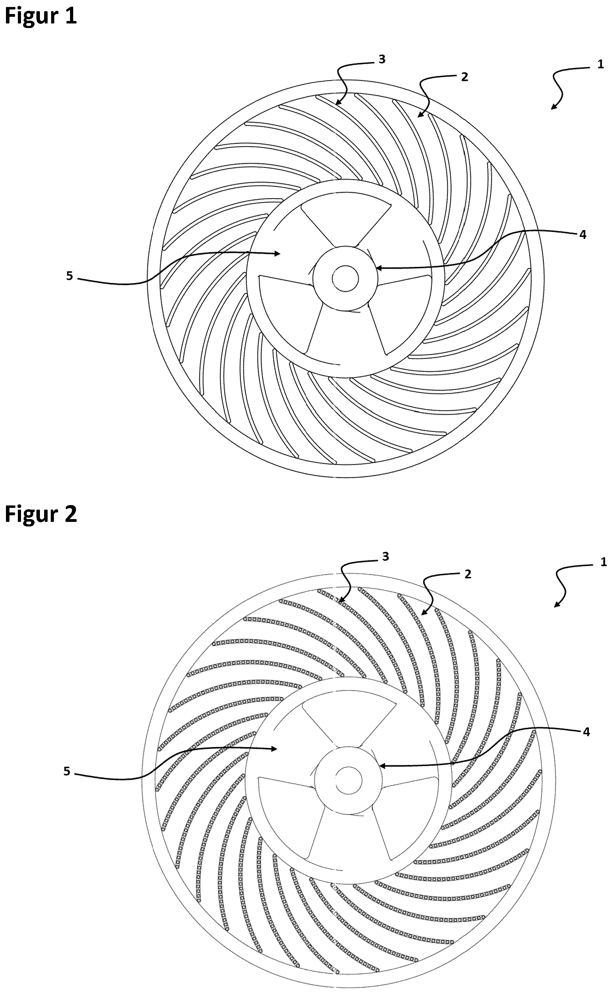

Die Figur 1 zeigt schematisch eine Ausgestaltung eines Gasaufnahmeraums 1 in der Aufsicht. Der Gasaufnahmeraum 1 ist zylindrisch ausgestaltet und unterteilt sich in einen inneren Bereich, welcher die Aufnahme für ein Prozessgas 4 und/oder eine Aufnahme für eine Welle 4 umfasst. Über diese Aufnahme 4 wird der Gasaufnahmeraum 1 sowohl mit Prozessgas wie auch mit mechanischer Antriebsenergie versorgt. Das Prozessgas wird von der Aufnahme 4 über die trapezförmigen Verbindungsstücke 5 in den eigentlichen Gasaufnahmeraum 1 geleitet. Der Gasaufnahmeraum 1 zeigt eine geschlossene Oberfläche 2, welche zur Aufnahme der Diffusionsmembranen (in dieser Figur nicht dargestellt) mit entsprechenden Aussparungen 3 versehen ist. In die Aussparungen 3 können die Diffusionsmembranen in Form von Hohlfasern eingeklemmt oder geklebt werden und die Diffusionsmembranen reichen in den Innenraum des Gasaufnahmeraumes 1 hinein, so dass eine durchgehende gasführende Strecke von der Aufnahme 4, über die Verbindungsstücke 5, in das eigentliche Innere des Gasaufnahmeraums 1 bis in die Hohlfasermembranen erfolgen kann. Die Aussparungen 3 können auch als Diffusionsmembranen-Fixierungsfläche 3 bezeichnet werden. Durch die Geometrie der Diffusionsmembranen-Fixierungsfläche 3 wird die flächige Ausgestaltung der Diffusionsmembranen festgelegt. In dieser Ausgestaltung weißt die Diffusionsmembranen-Fixierungsfläche 3 eine bogenförmige Ausgestaltung auf, mit der Konsequenz, dass die in diesen Aussparungen befestigten Hohlfasern in Summe eine ebenfalls gebogene Membranfläche aufweisen. Der obere und der untere Gasaufnahmeraum 1 können spiegelbildlich ausgestaltet sein. Es ist aber auch möglich, dass einer der beiden Gasaufnahmeräume 1 keine Aufnahme für weitere Prozessmittel aufweist. Im Betrieb können die Gasaufnahmeräume 1 durch Abdeckungen an der Unter- oder Oberseite vor einem direkten Zutritt von Prozessmitteln geschützt werden.

Die Figur 2 zeigt im Wesentlichen dieselbe Ausgestaltung wie die Figur 1 mit der Oberfläche des Gasaufnahmeraums 2, der Diffusionsmembranen-Fixierungsmittel 3, der Aufnahme für ein Prozessgas und/oder eine Welle 4, sowie die Anbindung 5 des Gasaufnahmeinnenraum mit der Gasaufnahme 4. Im Unterschied zu der Figur 1 ist an dieser Stelle angedeutet, dass einzelne zylindrische Hohlfasern in die Diffusionsmembranen-Fixierungsfläche 3 eingefügt sind. Somit wird an dieser Stelle verdeutlicht, dass die Fläche nicht eine durchgehende flächige Membran, sondern aus vielen einzelnen Hohlfasern gebildet wird, die sowohl in X- wie auch in Y-Richtung gegeneinander versetzt sind, so dass sie der bogenförmigen Ausgestaltung der Diffusionsmembranen-Fixierungsfläche 3 folgen. Insofern ergibt sich für die Diffusionsmembranen eine bogenförmige Geometrie. Neben der Fixierung der Hohlfasern in den beiden Gas-Aufnahmeräumen 1 können die einzelnen Hohlfasern auch noch gegeneinander mechanisch fixiert werden (in dieser Figur nicht dargestellt). Diese zusätzliche Fixierung kann beispielsweise über Fasern erfolgen, welche senkrecht zur Symmetrieachse der Hohlfasern in die Membran eingewebt oder eingeflochten sind. Es wird also ein Gitter aus Hohlfasern und den Fasern der zusätzlichen Fixierung gebildet, welches als Funktion der Anzahl der weiteren Fixierungspunkte und als Funktion der mechanischen Eigenschaften der zusätzlichen Fasern auf die nötige mechanische Belastbarkeit der Diffusionsmembranen abgestimmt werden kann.

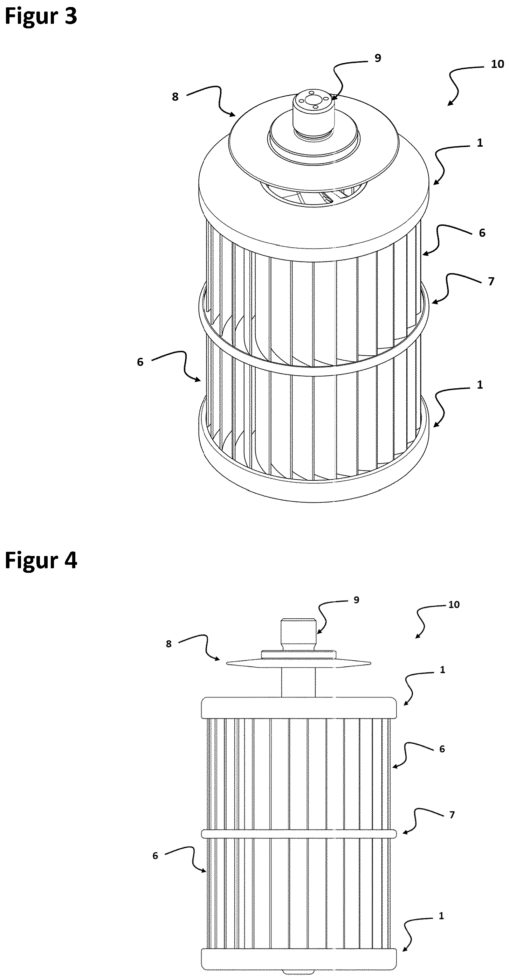

Die Figur 3 zeigt einer Ausgestaltung einer erfindungsgemäßen Begasungseinheit 10. In dieser Figur ist die gesamte Begasungseinheit 10 zu sehen. Die Begasungseinheit 10 wird über eine Zuleitung 9 mit den Prozessmedien Prozessgases und Energie versorgt. Die Zuleitung mündet in der Aufnahme 4 für die Gaszufuhr/Welle des ersten Gasaufnahmeraums 1 (nicht dargestellt). Der Gasaufnahmeraum 1 ist an dieser Stelle mit einer oberen Abdeckung versehen. Aus dem Gasaufnahmeraum 1 erstrecken sich die einzelnen Diffusionsmembranen 6, welche sich in Summe von dem ersten Gasaufnahmeraum 1 (hier oben dargestellt) bis zum unteren Gasaufnahmeraum 1 erstrecken. In dieser Figur lässt sich insbesondere die flächige Ausgestaltung der Diffusionsmembranen 6 erkennen, welche durch eine gezielte Anordnung von Hohlfasern erreicht wird. Die Diffusionsmembranen 6 erstrecken sich vom ersten bis hin zum zweiten Gasaufnahmeraum 1 und werden in der Mitte durch eine Haltescheibe 7 gehalten. Zwischen den Gasaufnahmeräumen 1 wird also die Gasleitung der einzelnen Diffusionsmembranen 6 nicht unterbrochen. Die Diffusionsmembranen 6 können durch die Haltescheibe 7 entweder nur gehalten, aus ihrer ursprünglichen Lage ausgelenkt oder mechanisch gespannt werden. Durch die Haltescheibe 7 kann also die Ausrichtung der einzelnen Diffusionsmembranen 6 geändert werden, welches natürlich einen Einfluss auf die erreichbare Konvektion der Begasungseinheit 10 ausübt. In dieser Figur ist zudem ein Strömungsbrecher 8 dargestellt, welcher oberhalb der Begasungseinheit 10 in Richtung Reaktorkopfraum angeordnet ist. Dieser Strömungsbrecher 8 ist optional und kann insbesondere bei sehr hohen Umlaufgeschwindigkeiten der Begasungseinheit 10 eine Vortexbildung in der Reaktorflüssigkeit verhindern. Dies kann zu einer weiteren Verringerung der Blasenbildung beitragen.

Die Figur 4 zeigt eine erfindungsgemäße Ausgestaltung einer Begasungseinheit 10 in der Frontansicht. Die Begasungseinheit 10 wird über eine Zuleitung 9 mit den Prozessmedien Prozessgases und mechanische Energie versorgt. Die Zuleitung mündet in der Aufnahme für die Gaszufuhr/Welle des ersten Gasaufnahmeraums 1 (nicht dargestellt). Aus dem Gasaufnahmeraum 1 erstrecken sich die einzelnen Diffusionsmembranen 6 welche sich in Summe vom oberen 1 bis zum unteren Gasaufnahmeraum 1 erstrecken. Die Diffusionsmembranen 6 erstrecken sich vom ersten 1 bis hin zum zweiten Gasaufnahmeraum 1 und werden in der Mitte durch eine Haltescheibe 7 gehalten. In dieser Figur ist zudem ein Strömungsbrecher 8 dargestellt, welcher oberhalb der Begasungseinheit 10 in Richtung Reaktorkopfraum angeordnet ist.

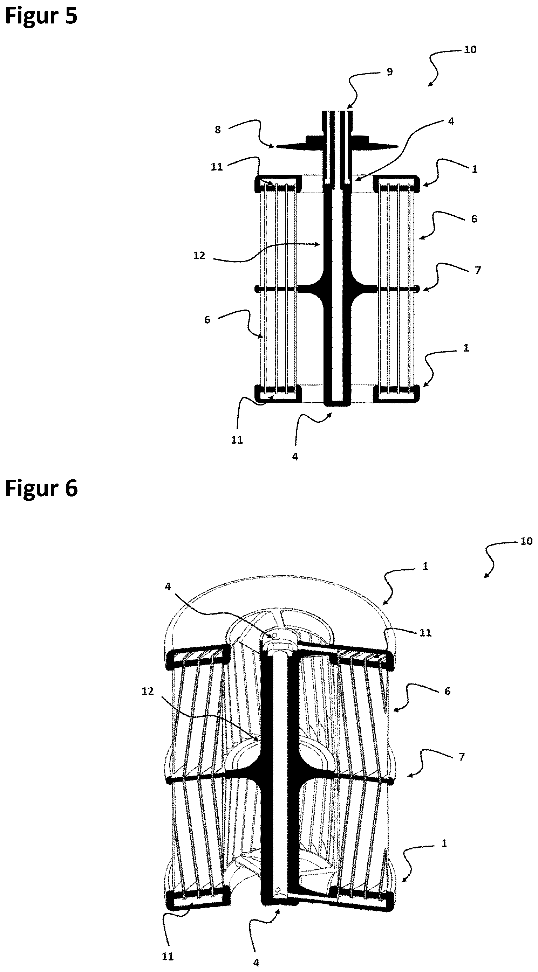

Die Figur 5 zeigt beispielhaft die Medienführung innerhalb einer erfindungsgemäßen Begasungseinheit 10. Die Begasungseinheit 10 wird durch eine Hohlwelle 9 angetrieben, welche an der Aufnahme 4 für die Gaszufuhr/Welle mit dem Gasaufnahmeraum 1 verbunden ist. Durch die Welle wird sowohl die mechanische Energie wie auch das Prozessgas an den Gasaufnahmeraum 1 transportiert. Über die Aufnahme 4 für die Gaszufuhr und die Anbindung Gasaufnahmeinnenraum-Gasaufnahme wird das Gas in den inneren Gasaufnahmeraum 11 geleitet. Die einzelnen Hohlfasermembranen 6 sind so am Gasaufnahmeraum 1 angeordnet, dass sie sich in den inneren Gasaufnahmeraum 11 erstrecken. Die Hohlfasermembranen werden also durch den inneren Gasaufnahmeraum 11 mit Prozessgas versorgt, welches durch die Hohlfasern der Diffusionsmembranen 6 in den anderen Gasaufnahmeraum 1 geleitet wird. Das Prozessgas kann aus den Hohlfasern der Diffusionsmembranen 6 in die Prozessflüssigkeit übertreten und so die Flüssigkeit mit Prozessgas versorgen. Beide Gasaufnahmeräume 1 sind dabei zusätzlich noch über eine mechanische Stütze 12 miteinander verbunden. Diese Stütze kann neben der rein mechanischen Stützung auch noch für weitere technische Funktionen verwendet werden. In dieser Ausführungsform umfasst auch der zweite (untere) Gasaufnahmeraum 1 eine Aufnahme 4 für das Prozessgas. Das nicht aus den Membranen 6 diffundierte Prozessgas wird über die Stütze 12 in Form einer Hohlwelle aus der Begasungseinheit 10 geführt. Die Begasungseinheit 10 wird also in einer "cross-flow" Arbeitsweise betrieben. Generell kann also die Ab- ebenso wie die Zufuhr des Prozessgases über eine zentrale Hohlwelle 12 erfolgen, welche auch als mechanische Stütze fungieren kann. Insbesondere letztere Ausgestaltung kann die Anzahl an nötigen Verbindungsstellen reduzieren.

Die Figur 6 zeigt, ebenfalls wie die Figur 5, die Medienführung innerhalb einer erfindungsgemäßen Begasungseinheit 10. Die Begasungseinheit 10 wird durch die Aufnahme 4 mit Prozessgas und/oder mechanischer Energie versorgt. Die Aufnahme 4 ist mit dem Gasaufnahmeraum 1 verbunden, wobei das Prozessgas in den inneren Gasaufnahmeraum 11 geleitet wird. Die einzelnen Hohlfasermembranen 6 sind am Gasaufnahmeraum 1 angeordnet und erstrecken sich in diesen. Die Hohlfasermembranen 6 werden also durch den inneren Gasaufnahmeraum 11 mit Prozessgas versorgt, welches durch die Hohlfasern der Diffusionsmembranen 6 in den anderen Gasaufnahmeraum 1 geleitet wird. Das Prozessgas kann aus den Hohlfasern der Diffusionsmembranen 6 in die Prozessflüssigkeit übertreten und so die Flüssigkeit mit Prozessgas versorgen. Beide Gasaufnahmeräume 1 sind dabei zusätzlich noch über eine mechanische Stütze 12 miteinander verbunden, welche optional ebenfalls Prozessgas führen kann. In dieser Ausführungsform umfasst auch der zweite (untere) Gasaufnahmeraum 1 eine Aufnahme 4 für das Prozessgas. Das nicht aus den Membranen 6 diffundierte Prozessgas wird über eine Gasleitung in der mechanischen Stütze 12 aus der Begasungseinheit 10 geführt. Die Begasungseinheit 10 kann in dieser Ausgestaltung in einer "cross-flow" Arbeitsweise betrieben werden. Generell kann also die Ab- ebenso wie die Zufuhr 4 des Prozessgases über eine zentrale mechanische Stütze 12 in Form einer Hohlwelle erfolgen. Insbesondere letztere Ausgestaltung kann die Anzahl an nötigen Verbindungsstellen reduzieren.

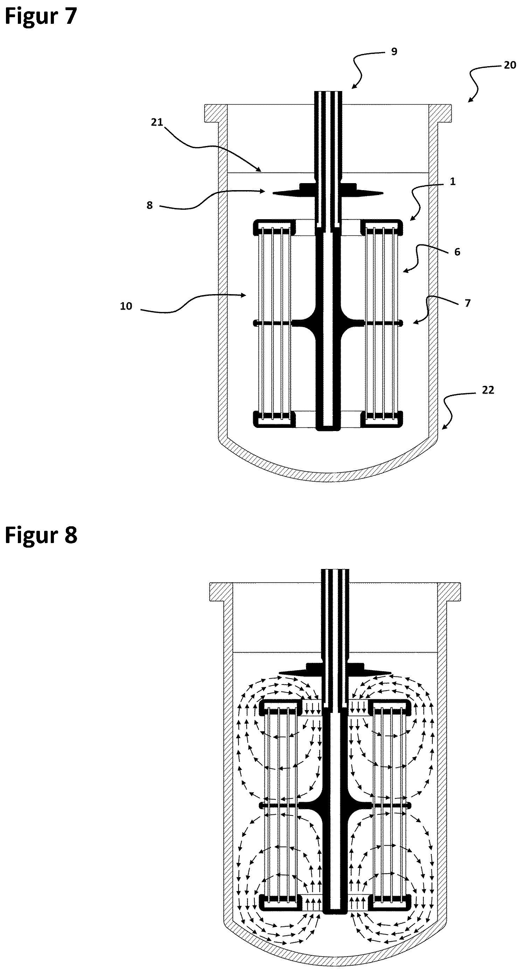

Die Figur 7 zeigt einen erfindungsgemäßen Bioreaktor 20 mit der darin befindlichen, erfindungsgemäßen Begasungseinheit 10. Der Reaktor 20 ist mit einer Prozessflüssigkeit gefüllt, wobei die Prozessflüssigkeit bis zum Prozessflüssigkeitsspiegel 21 im Reaktor 20 vorliegt. Die Begasungseinheit 10 wird durch die Prozessgas-/mechanische Energiezufuhr in Form einer Hohlwelle 9 im Reaktor 20 gehalten und durch diesen bewegt. Der weitere Aufbau der Begasungseinheit 10 kann der Beschreibung zur Figur 5 entnommen werden. Es ist des Weiteren möglich, dass an den Reaktorwänden 22 oder im Flüssigkeitsvolumen des Reaktors 20 noch weitere Strömungsbrecher oder -Leiter 8 angeordnet sind (in dieser Figur nicht dargestellt), wobei mit diesem weiteren Strömungsbrecher 8 die Konvektion der Prozessflüssigkeit beeinflusst werden kann.

Die Figur 8 zeigt eine Möglichkeit zur Ausgestaltung eines erfindungsgemäßen Reaktors 20 mit erfindungsgemäßer Begasungseinheit 10. In dieser Figur ist eine Möglichkeit zur Ausbildung von Konvektionsströmungen innerhalb des Reaktors 20 dargestellt. Die Konvektionsströmungen ergeben sich aus einer Simulation des Strömungsverhalten in Abhängigkeit der Geometrie des Reaktors 20 und der Begasungseinheit 10. In dieser Figur ist zu erkennen, dass die Begasungseinheit 10 zur Ausbildung eines sehr symmetrischen Konvektionsstromes führt, wobei insbesondere auch das Innere der Begasungseinheit 10 von der Prozessflüssigkeit aktiv umströmt wird. Insbesondere Letzteres kann dazu beitragen, dass ein besonders effizienter Eintrag von Prozessgas durch die Diffusionsmembranen 6 in die Prozessflüssigkeit erfolgen kann. Durch die Konvektion der Prozessflüssigkeit um die einzelnen Hohlfasern 6 werden insbesondere kleine Gasbläschen gebildet, welche von der Oberfläche der Hohlfasern 6 abgeschert werden und so die Prozessflüssigkeit mit Prozessgas versorgen können. Auf diese Art und Weise wird auch sichergestellt, dass die Blasengröße an der Oberfläche der Hohlfasern klein gehalten wird.

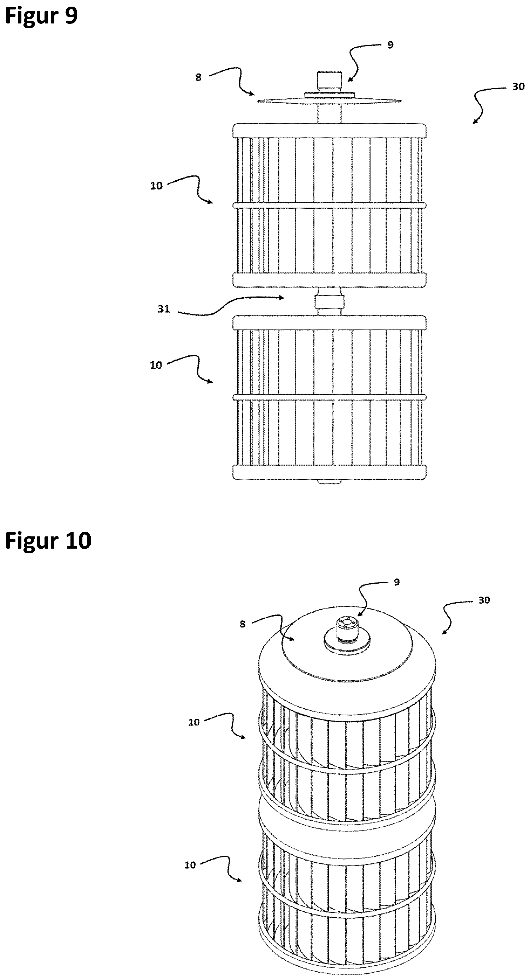

Die Figur 9 zeigt eine seriell geschaltete Begasungseinheit 30 aus zwei einzelnen Begasungseinheiten 10, welche über eine Modul-Kupplung 31 gekoppelt sind. Über die Modul-Kupplung 31 wird sowohl das Prozessgas wie auch die mechanische Bewegung von der oberen Begasungseinheit 10 auf die untere Begasungseinheit 10 übertragen. Durch das Zusammenschalten mehrerer Begasungseinheiten 10 lassen sich unterschiedliche Reaktorgeometrien und auch -Größen sehr effizient mit Prozessgas versorgen. Es ergibt sich ein Aufbau mit möglichst wenigen Anschlüssen und einer verlässlich vorhersehbaren Konvektionsströmung. Somit können gerade über seriell geschaltete Begasungseinheiten 30 leicht Up-scalings auf grö-ßere Reaktoren 20 durchgeführt werden.

Die Figur 10 zeigt die Ausgestaltung der Figur 9 aus einer anderen Perspektive.

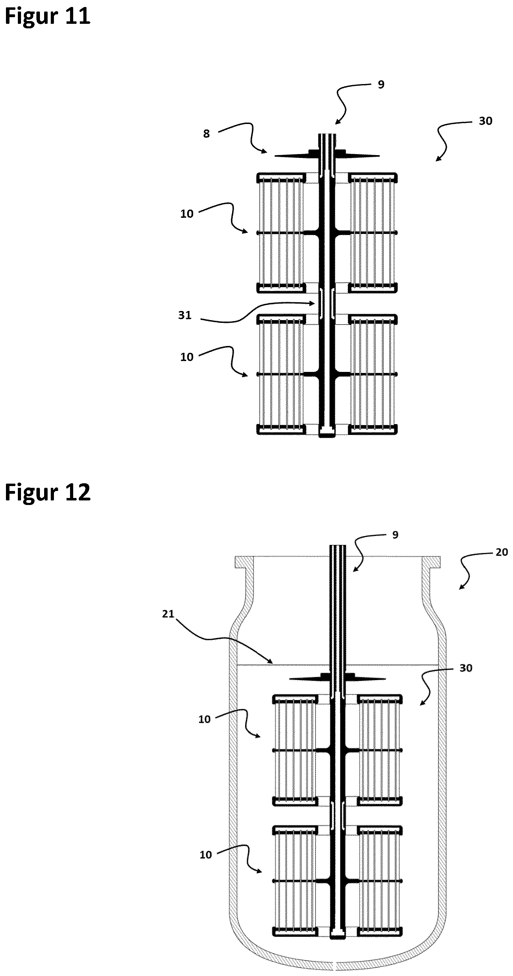

Die Figur 11 zeigt beispielhaft die Medienführung innerhalb einer Anordnung aus zwei seriell geschalteten Begasungseinheiten 10. Die Begasungseinheit 10 wird durch eine Hohlwelle 9 angetrieben, welche an der Aufnahme für die Gaszufuhr/Welle 4 mit dem Gasaufnahmeraum 1 verbunden ist. Über die Aufnahme für die Gaszufuhr 4 und die Anbindung Gasaufnahmeinnenraum-Gasaufnahme 5 wird das Gas in über die einzelnen Hohlfasermembranen 6 in den anderen Gasaufnahmeraum 1 geleitet. Aus diesem zweiten Gasaufnahmeraum 1 kann das restliche Prozessgas über eine Modul-Kupplung 31 in die zweite Begasungseinheit 10 übergehen. Die Modul-Kupplung 31 zwischen den beiden Begasungseinheiten 10 ermöglicht dabei sowohl den Transfer des Prozessgases wie auch die Weitergabe der mechanischen Antriebsenergie zwischen den einzelnen Begasungseinheiten 10.

Die Figur 12 zeigt eine Ausgestaltung eines erfindungsgemäßen Reaktors 20 mit zwei seriell geschalteten Begasungseinheiten 30, welche aus zwei einzelnen Begasungseinheiten 10 bestehen. Über das serielle Zusammenschalten mehrerer Begasungseinheiten 10 lassen sich insbesondere auch Reaktoren 20 mit einem großen Aspektverhältnis sicher mit Prozessgas versorgen.

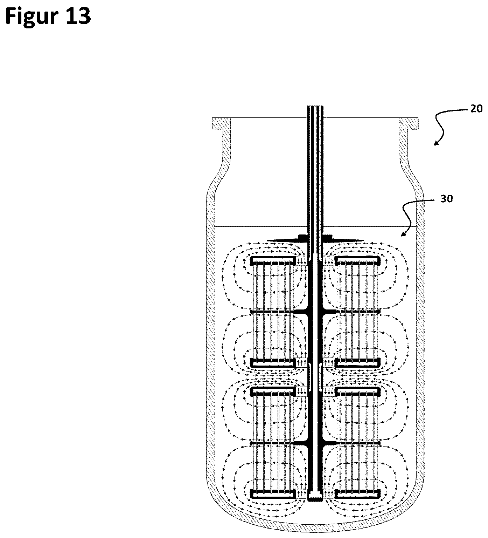

Die Figur 13 zeigt ein mögliches Strömungsprofil eines Reaktors 20, welcher mit zwei seriell geschalteten Begasungseinheiten 30 ausgestattet wurde. Es ergibt sich eine gleichmäßige Konvektion der Prozessflüssigkeit sowohl über den gesamten Reaktorbereich als auch innerhalb der seriell geschalteten Begasungseinheit 30.

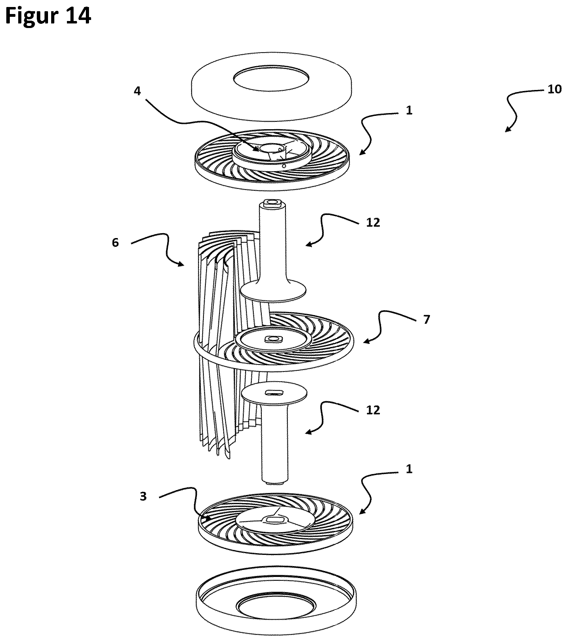

Die Figur 14 zeigt eine erfindungsgemäße Begasungseinheit 10 in einer Explosionsansicht. Die Begasungseinheit 10 kann beispielsweise durch eine Hohlwelle (in dieser Figur nicht dargestellt) angetrieben und mit Prozessgas versorgt werden, welche an der Aufnahme 4 mit dem Gasaufnahmeraum 1 verbunden ist. Durch die Welle wird sowohl die mechanische Energie der Einheit bereitgestellt, wie auch das Prozessgas an den Gasaufnahmeraum 1 transportiert. Die einzelnen Hohlfasermembranen 6 sind so am Gasaufnahmeraum 1 angeordnet, dass sie sich in den inneren Gasaufnahmeraum 11 erstrecken. Die Hohlfasermembranen werden also durch den inneren Gasaufnahmeraum 11 mit Prozessgas versorgt, welches durch die Hohlfasern der Diffusionsmembranen 6 in den anderen (unteren) Gasaufnahmeraum 1 geleitet wird. Das Prozessgas kann aus den Hohlfasern der Diffusionsmembranen 6 in die Prozessflüssigkeit übertreten und so die Flüssigkeit mit Prozessgas versorgen. Beide Gasaufnahmeräume 1 sind dabei zusätzlich noch über eine mechanische Stütze 12 miteinander verbunden. Diese Stütze 12 kann neben der rein mechanischen Stützung auch noch für weitere technische Funktionen verwendet werden. In dieser Ausführungsform umfasst auch der zweite (untere) Gasaufnahmeraum 1 eine Aufnahme 4 für Prozessgas. Das nicht aus den Membranen 6 diffundierte Prozessgas wird über die Hohlwelle der mechanischen Stütze 12 aus der Begasungseinheit 10 wieder herausgeführt. Die Begasungseinheit 10 wird in dieser Ausgestaltung also in einer "cross-flow" Arbeitsweise betrieben. Generell kann also die Ab- ebenso wie die Zufuhr des Prozessgases über eine zentrale Hohlwelle in der mechanischen Stütze 12 erfolgen, welche an einem oder beiden Gasaufnahmeräumen 1 über Aufnahmen 4 an die äußere Peripherie angeschlossen sind. Insbesondere letztere Ausgestaltung kann die Anzahl an nötigen Verbindungsstellen reduzieren und zu einer kompakten Bauform beitragen. Zudem ist in dieser Ausgestaltung zu erkennen, dass die einzelnen Gasaufnahmeräume 1 ober und unterhalb durch Abdeckscheiben geschützt ausgeführt werden können.

### Bezugszeichen

- 1: Gasaufnahmeraum
- 2: Oberfläche Gasaufnahmeraum
- 3: Diffusionsmembranen-Fixierungsfläche
- 4: Aufnahme für Gaszufuhr/Welle
- 5: Anbindung Gasaufnahmeinnenraum - Gasaufnahme
- 6: Diffusionsmembranen
- 7: Haltescheibe
- 8: Strömungsbrecher
- 9: Prozessgas-/mechanische Energiezufuhr
- 10: Begasungseinheit
- 11: Innerer Gasaufnahmeraum
- 12: Stütze
- 20: Gas-Flüssig-Reaktor
- 21: Prozessflüssigkeitsspiegel
- 22: Reaktorhülle
- 30: Seriell geschaltete Begasungseinheiten
- 31: Modul-Kupplung

## Patentansprüche

1. Begasungseinheit (10) zum blasenfreien Eintrag eines Prozessgases in eine in einem Reaktor (20) befindliche Flüssigkeit, **dadurch gekennzeichnet, dass** die Begasungseinheit (10) mindestens umfasst:
- einen ersten und, von diesem beabstandet, einen zweiten Gasaufnahmeraum (1) zur Aufnahme eines Prozessgases, wobei beide Gasaufnahmeräume (1) über mindestens zwei flächig ausgebildete, gasführende Diffusionsmembranen (6) aus voneinander beabstandeten und zumindest partiell gegeneinander fixierten Hohlfasern (6) miteinander verbunden sind;
- eine Aufnahme für eine Gaszufuhr (4) an mindestens einem der Gasaufnahmeräume (1);
- eine Aufnahme (4) für eine Welle (9) an mindestens einem der Gasaufnahmeräume (1);
wobei die Begasungseinheit (10) zur Begasung der Flüssigkeit im Reaktor (20) über die Gaszufuhraufnahme (4) mit Prozessgas versorgbar, über die Aufnahme (4) für die Welle (9) in eine Rotationsbewegung versetzbar und über die Rotationsbewegung der Begasungseinheit (10) in der Flüssigkeit eine Konvektionsströmung innerhalb des Reaktors (20) ausbildbar ist.

2. Begasungseinheit nach Anspruch 1, wobei die Projektionen der Diffusionsmembranen (6) auf die Aufnahmeräume (1) eine Kreisbogengeometrie aufweisen.

3. Begasungseinheit nach einem der vorhergehenden Ansprüche, wobei die Aufnahme für die Gaszufuhr (4) und die Aufnahme (4) für die Welle (9) an nur einem Gasaufnahmeraum (1) angeordnet sind.

4. Begasungseinheit nach einem der vorhergehenden Ansprüche, wobei die beiden Gasaufnahmeräume (1) jeweils zylindrisch ausgebildet und über eine oder mehrere mechanische Stützen (12) miteinander verbunden sind.

5. Begasungseinheit nach Anspruch 4, wobei zwischen den beiden Gasaufnahmeräumen (1) an der mechanischen Stütze (12) mindestens eine Haltescheibe (7) angeordnet ist, welche dazu eingerichtet ist die Diffusionsmembranen (6) mechanisch zu halten.

6. Begasungseinheit nach einem der Ansprüche 4 oder 5, wobei die mechanische Stütze (12) dazu eingerichtet ist Prozessgas aus den Gasaufnahmeräumen (1) zu transportieren.

7. Begasungseinheit nach einem der vorhergehenden Ansprüche, wobei das Flächenverhältnis aus gesamter Hohlfaser-Querschnittsfläche zur Querschnittsfläche des Gasaufnahmeraumes größer oder gleich 5 % und kleiner oder gleich 45% beträgt.

8. Begasungseinheit nach einem der vorhergehenden Ansprüche, wobei die Packungsdichte der Diffusionsmembranen (6) bezogen auf das Volumen der Begasungseinheit (10), ausgedrückt als Oberfläche der Hohlfasern dividiert durch das Volumen der Begasungseinheit (10), größer oder gleich 0,1 cm⁻¹ und kleiner oder gleich 7,5 cm⁻¹ beträgt.

9. Verfahren zur Begasung einer Prozessflüssigkeit innerhalb eines Reaktors (20), **dadurch gekennzeichnet, dass** der Gaseintrag über eine Begasungseinheit (10) nach einem der Ansprüche 1 - 8 erfolgt.

10. Verfahren nach Anspruch 9, wobei die Umlaufgeschwindigkeit der Membranfläche (6) am äußersten Rand der Begasungseinheit (10) größer oder gleich 0,1 m/s und kleiner oder gleich 5 m/s beträgt.

11. Gas-Flüssig-Reaktor (20) mindestens umfassend eine äußere Reaktorhülle (22), eine Antriebseinheit, eine Gaszufuhr (9) und eine Begasungseinheit (10) nach einem der Ansprüche 1-8.

12. Gas-Flüssig-Reaktor nach Anspruch 11, wobei der Reaktor (20) neben der Begasungseinheit (10) keine weitere Rühreinheit aufweist.

13. Gas-Flüssig-Reaktor nach Anspruch 12, wobei zwischen der Reaktorhülle (22) und einem Gasaufnahmeraum (1) mindestens ein Strömungsbrecher (8) angeordnet ist.

14. Verwendung eines Gas-Flüssig-Reaktors (20) nach einem der Ansprüche 11 - 13 zur Versorgung in einer Prozesslösung suspendierter oder an dem Reaktorinneren oder an der Begasungseinheit anhaftender biologischer Kulturen mit Prozessgasen.

15. Verwendung nach Anspruch 14, wobei die biologischen Kulturen dazu eingerichtet sind schaumbildende Substanzen herzustellen.

## Claims

1. A gassing unit (10) for bubble-free introduction of a process gas into a liquid located in a reactor (20), **characterized in that** the gassing unit (10) at least comprises:
- a first gas receiving chamber and, spaced therefrom, a second gas receiving chamber (1) for receiving a process gas, the two gas receiving chambers (1) being connected to one another via at least two two-dimensional, gas-conducting diffusion membranes (6) comprising hollow fibers (6) spaced apart from one another and at least partially fixed to one another;
- a receptacle for a gas supply (4) on at least one of the gas receiving chambers (1);
- a receptacle (4) for a shaft (9) on at least one of the gas receiving chambers (1);
wherein the gassing unit (10) for gassing the liquid in the reactor (20) can be supplied with process gas via the gas supply receptacle (4), can be set into a rotational movement via the receptacle (4) for the shaft (9) and can form a convection flow within the reactor (20) via the rotational movement of the gassing unit (10) in the liquid.

2. Gassing unit according to claim 1, wherein the projections of the diffusion membranes (6) onto the gas receiving chambers (1) have a circular arc geometry.

3. Gassing unit according to any one of the preceding claims, wherein the receptacle for the gas supply (4) and the receptacle (4) for the shaft (9) are arranged at only one gas receiving chamber (1).

4. Gassing unit according to any one of the preceding claims, wherein the two gas receiving chambers (1) are each of cylindrical shape and are interconnected via one or more mechanical supports (12).

5. Gassing unit according to claim 4, wherein at least one retaining disk (7) is arranged between the two gas receiving chambers (1) on the mechanical support (12), which is set up to mechanically retain the diffusion membranes (6).

6. Gassing unit according to any one of claims 4 or 5, wherein the mechanical support (12) is adapted to transport process gas from the gas receiving chambers (1).

7. Gassing unit according to any one of the preceding claims, wherein the area ratio of total hollow fiber cross-sectional area to the cross-sectional area of the gas receiving chamber is greater than or equal to 5% and less than or equal to 45%.

8. Gassing unit according to any one of the preceding claims, wherein the packing density of the diffusion membranes (6) relative to the volume of the gassing unit (10), expressed as the surface area of the hollow fibers divided by the volume of the gassing unit (10), is greater than or equal to 0.1 cm⁻¹ and less than or equal to 7.5 cm⁻¹

9. Method for gassing a process liquid within a reactor (20), **characterized in that** the gas input is effected by a gassing unit (10) according to any one of claims 1 - 8.

10. The method of claim 9, wherein the rotational speed of the membrane surface (6) at the outermost edge of the gassing unit (10) is greater than or equal to 0.1 m/s and less than or equal to 5 m/s.

11. Gas-liquid reactor (20) at least comprising an outer reactor shell (22), a drive unit, a gas supply (9) and a gassing unit (10) according to any one of claims 1-8.

12. Gas-liquid reactor according to claim 11, wherein the reactor (20) does not comprise a stirring unit other than the gassing unit (10).

13. Gas-liquid reactor according to claim 12, wherein at least one flow breaker (8) is arranged between the reactor shell (22) and a gas receiving chamber (1).

14. Use of a gas-liquid reactor (20) according to any one of claims 11 - 13 for supplying process gases to biological cultures suspended in a process solution or adhering to the reactor interior or to the gassing unit.

15. Use according to claim 14, wherein the biological cultures are adapted to produce foam-forming substances.

## Revendications

1. Unité de gazage (10) pour l'introduction sans bulles d'un gaz de procédé dans un liquide situé dans un réacteur (20), **caractérisée en ce que** l'unité de gazage (10) comprend au moins:
- un premier et, à distance de celui-ci, un deuxième espace de réception de gaz (1) destiné à recevoir un gaz de procédé, dans laquelle les deux espaces de réception de gaz (1) sont reliés entre eux par au moins deux membranes de diffusion plates transportant du gaz (6) constituées de fibres creuses (6) espacées l'une de l'autre et au moins partiellement fixées l'une contre l'autre;
- une entrée d'alimentation en gaz (4) sur au moins l'un des espaces de réception de gaz (1);
- une entrée (4) pour arbre (9) sur au moins l'un des espaces de réception de gaz (1);
dans laquelle l'unité de gazage (10) pouvant être alimentée en gaz de procédé pour le gazage du liquide dans le réacteur (20) via l'entrée d'alimentation en gaz (4), pouvant être mise en mouvement de rotation par l'entrée (4) pour l'arbre (9) et un courant de convection pouvant être formé dans le liquide à l'intérieur du réacteur (20) par le mouvement de rotation de l'unité de gazage (10).

2. Unité de gazage selon la revendication 1, dans laquelle les projections des membranes de diffusion (6) sur les espaces de réception (1) présentent une géométrie d'arc circulaire.

3. Unité de gazage selon l'une des revendications précédentes, dans laquelle l'entrée d'alimentation en gaz (4) et l'entrée (4) pour l'arbre (9) sont disposées sur un seul espace de réception du gaz (1).

4. Unité de gazage selon l'une des revendications précédentes, dans laquelle les deux espaces de réception du gaz (1) sont chacun cylindriques et sont reliés entre eux par un ou plusieurs supports mécaniques (12).

5. Unité de gazage selon la revendication 4, dans laquelle au moins un disque de retenue (7) est disposé entre les deux espaces de réception du gaz (1) sur le support mécanique (12), conçu pour maintenir mécaniquement les membranes de diffusion (6).

6. Unité de gazage selon l'une des revendications 4 ou 5, laquelle le support mécanique (12) est conçu pour transporter le gaz de procédé depuis les espaces de réception de gaz (1).

7. Unité de gazage selon l'une des revendications précédentes, dans laquelle le rapport entre la surface totale de section transversale de fibres creuses et la surface de section transversale de l'espace de réception de gaz est supérieur ou égal à 5 % et inférieur ou égal à 45 %.

8. Unité de gazage selon l'une des revendications précédentes, dans laquelle la densité de remplissage des membranes de diffusion (6) par rapport au volume de l'unité de gazage (10), exprimée en surface de fibres creuses divisée par le volume de l'unité de gazage (10), est supérieure ou égale à 0,1 cm⁻¹ et inférieur ou égal à 7,5 cm⁻¹

9. Procédé de gazage d'un liquide de procédé à l'intérieur d'un réacteur (20), **caractérisée en ce que** l'entrée de gaz est effectuée via une unité de gazage (10) selon l'une des revendications 1 à 8.

10. Procédé selon la revendication 9, dans lequel la vitesse de circulation de la surface de la membrane (6) au niveau du bord le plus extérieur de l'unité de gazage (10) est supérieure ou égale à 0,1 m/s et inférieure ou égale à 5 m/s.

11. Réacteur gaz-liquide (20) comprenant au moins une enveloppe externe de réacteur (22), une unité d'entraînement, une alimentation en gaz (9) et une unité de gazage (10) selon l'une quelconque des revendications 1 à 8.

12. Réacteur gaz-liquide selon la revendication 11, dans lequel le réacteur (20) ne comporte pas d'unité d'agitation supplémentaire en plus de l'unité de gazage (10).

13. Réacteur gaz-liquide selon la revendication 12, dans lequel au moins un brise-courant (8) est disposé entre l'enveloppe du réacteur (22) et un espace de réception de gaz (1).

14. Utilisation d'un réacteur gaz-liquide (20), selon l'une des revendications 11 à 13, pour l'alimentation en gaz de procédé de cultures biologiques en suspension dans une solution de traitement ou adhérant à l'intérieur du réacteur ou à l'unité de gazage.

15. Utilisation selon la revendication 14, dans laquelle les cultures biologiques sont conçues pour produire des substances moussantes.
